Europäisches Patentamt

European Patent Office

Office européen des brevets

(11) Publication number: **0 171 404**
**B1**

# EUROPEAN PATENT SPECIFICATION

(45) Date of publication of patent specification: **27.07.88**

(51) Int. Cl.⁴: **A 61 F 5/448**

(21) Application number: **85900707.2**

(22) Date of filing: **24.01.85**

(86) International application number:
**PCT/GB85/00035**

(87) International publication number:
**WO 85/03427 15.08.85 Gazette 85/18**

(54) OSTOMY APPLIANCE.

(30) Priority: **07.02.84 GB 8403237**

(43) Date of publication of application:
**19.02.86 Bulletin 86/08**

(45) Publication of the grant of the patent:
**27.07.88 Bulletin 88/30**

(84) Designated Contracting States:
**AT BE CH DE FR LI NL SE**

(56) References cited:
**EP-A-0 089 138**
**EP-A-0 142 259**
**FR-A-1 566 496**
**GB-A-2 115 288**
**US-A-3 814 278**
**US-A-3 865 109**
**US-A-4 359 051**

(73) Proprietor: **EDWARDS, John Victor**
**25 Furzefield Crescent**
**Reigate Surrey RH2 7HQ (GB)**

(72) Inventor: **EDWARDS, John Victor**
**25 Furzefield Crescent**
**Reigate Surrey RH2 7HQ (GB)**

(74) Representative: **Warren, Francis Charles et al**
**Baron & Warren 18 South End Kensington**
**London W8 5BU (GB)**

Courier Press, Leamington Spa, England.

# Description

The present invention relates to ostomy appliances and more particularly to a coupling for connecting an ostomy pouch to a pad which is adhesively attached to the abdominal skin of a user such that it surrounds the stoma. Such couplings have to be readily engageable and releasable in order to allow the pouch to be simple to fit to the user and for a soiled pouch to be easily replaced when necessary. Clearly, the coupling must be sufficiently secure so that the pouch does not become inadvertently detached from the user and must also be leakproof so that it does not allow leakage either of the liquid contents of the bag or of odours.

Numerous forms of coupling have been proposed by means of which an ostomy pouch may be attached to a user and whilst great advances have been made, particularly in recent years, in the production of coupling devices which are both more efficient and easy to use, as well as being more comfortable for the user, a number of disadvantages still exist with the coupling devices at present available. One such disadvantage is that it is possible for the coupling to become leaky or disengaged during the movement of the user so that the waste contents in the pouch escape on to the clothes or body of the user thereby giving rise to considerable inconvenience, embarrassment and discomfort. In order to reduce the risk of such happenings, it is known to provide a body belt which fits around the waist of the user and assists in holding the pouch in position. However with present constructions employing a body belt, the pouch is held in a fixed position on the users body which may be uncomfortable or awkward when the body is in certain positions, for example seated, thereby causing a user considerable discomfort.

It is an object of the present invention to provide an ostomy appliance and more particularly a form of coupling which seeks to reduce the aforementioned disadvantages.

It is known from FR-A-1 566 496 to provide a coupling for an ostomy appliance comprising two generally cylindrical rings which can be interengaged concentrically, the inner ring having means for attachment to an abdominal pad and the outer ring having means for attachment to an ostomy pouch, said inner ring including a radially outwardly directed peripheral bead and said outer ring including a resilient lip extending from the radially inner surface thereof whereby said lip becomes engaged behind said bead when the outer ring is fitted over the inner ring to make the coupling.

The present invention is characterised in that the outer ring attached to the pouch is fitted with a belt attaching ring which is mounted on said outer ring so as to be freely rotatable thereon and which is provided with attachment means to which the ends of a belt fitted round the waist of a user may be secured.

The invention also provides an ostomy pouch including an aperture for the passage of the stoma and surrounded by a ring forming the outer element of a concentric coupling for coupling the pouch to a pad attachable to the abdomen of a user, about the stoma wherein said ring includes a resilient lip extending from the radially inner surface of said ring and the free end of said lip faces towards the aperture in said pouch, characterised in that said ring carries a freely rotatable belt attaching ring mounted thereon and said belt attaching ring is provided with means for the attachment of the ends of a body belt fitted around the waist of a user.

It is to be understood that the term "ring" as used herein is intended to cover other closed figures besides truly circular figures and that the term "radial" should be construed accordingly.

According to one form of the invention, the belt attaching ring is also axially slidable on the outer ring attached to the pouch to a position whereat it assists in clamping the coupling parts in the interengaged position.

The belt-attaching ring may be provided with diametrically spaced attachment means, such as apertured lugs, by means of which the ends of the body belt may be attached to the ring.

It will be appreciated that since the belt-attaching ring is rotatable, it is readily possible for a user to orient or adjust the position of the pouch relative to the belt so that it is in the most comfortable position, without imposing any twist or strain on the belt.

The invention will now be further described by way of example, with reference to the accompanying drawings, in which:-

Figure 1 is a cross-sectional view through an ostomy appliance incorporating one embodiment of coupling according to this invention,

Figure 2 is an elevation of one embodiment of ostomy pouch,

Figure 3 is a side view partly in section of a further embodiment of ostomy pouch,

Figure 4 is a cross-sectional view through a further embodiment of ostomy coupling according to this invention; and

Figures 5A and 5B are respective plan views of the two parts of the coupling shown in Figure 4.

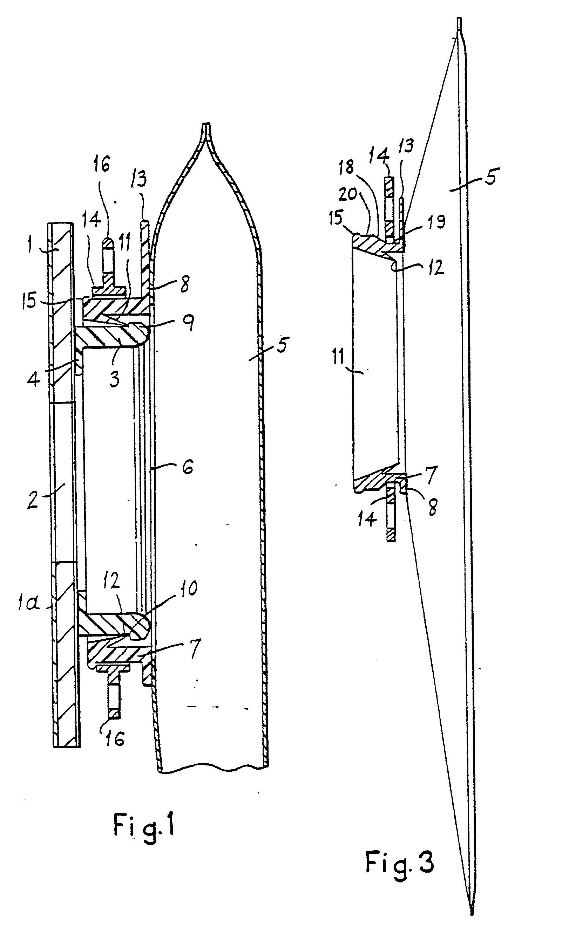

Referring to Figure 1, an ostomy appliance comprises a pad 1 for attachment to the skin of a user about the stoma which can project through an aperture 2 in the pad, which aperture is also surrounded by a ring 3 attached to the pad by a flange portion 4. The appliance also includes a pouch 5 having an aperture 6 therein for passage of the stoma and which is surrounded by a ring 7 attached to the periphery of the aperture 6 by means of a flange 8. The two rings 3 and 7 form the components of a coupling which can be interengaged to a secure locked position so as to hold the pouch 5 in its operative location, but which are also readily releasable when the pouch 5 is to be removed. More specifically, the ring 3 attached to the pad includes a peripheral bead 9 on the radially outer surface of the ring, this bead having or defining a latching surface formed by an outwardly extending wall portion 10, substantially perpendicular to the radially outer surface of the ring 3. The ring 7 attached to the pouch 5 includes a resilient tapered lip 11 extending from the radially inner surface of the ring, the free end of the lip facing towards the pouch tapering to an edge 12 which, as shown in Figure 1, engages the wall portion 10 behind the bead 9 when the two parts of the coupling are fitted together. More specifically, the two parts of the coupling are engaged by fitting the ring 7 on the pouch over the rim of the bead 9 on the ring 3 attached to the pad such that the lip 11 is deflected as it passes over the periphery of the bead and so that the free end 12 of the lip becomes engaged with the wall portion 10.

The two parts of the coupling can be readily disengaged by pulling the ring 7 so that the lip 11 is flexed and will slip back over the bead 9 on the ring 3. To assist in this operation, the ring 7 is provided with a pull tab 13.

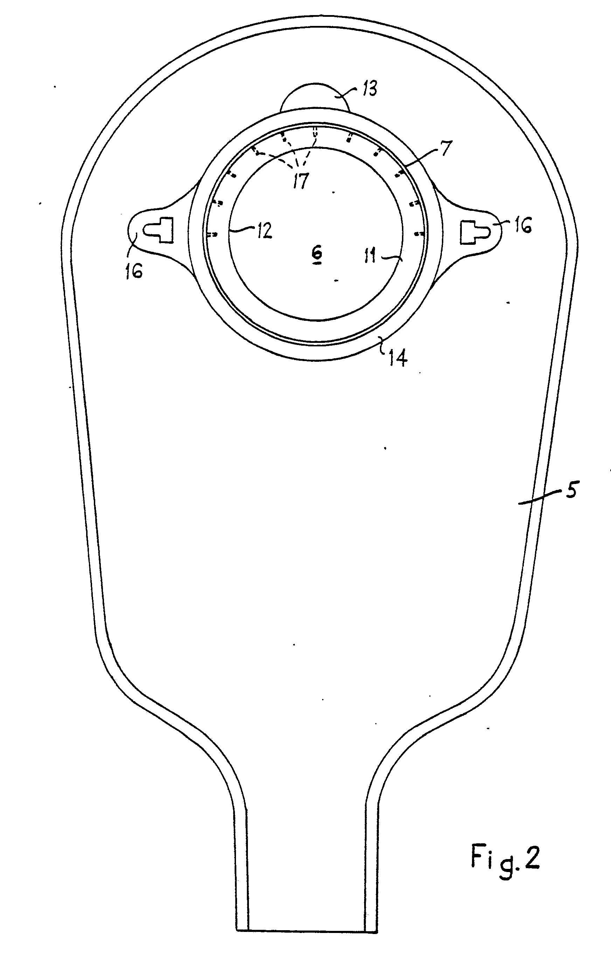

As seen more clearly in Figure 2 the coupling ring 7 attached to the pouch 5 may be fitted with an outer belt-attaching ring 14 which is mounted on the ring so as to be rotatable thereon and which is fitted onto the ring 7 by passing it over an outwardly directed peripheral bead 15. This belt-attaching ring 14 is provided with two diametrically spaced apertured lugs 16 into which opposite ends of a belt fitted round the waist of a user may be secured. The ring 14 is also axially slidable on the ring 7 to the position as shown in Figure 1 whereat it overlies the lip 11 and assists in clamping this lip in its engaged position behind the bead 9.

This Figure also indicates in dotted lines the position of a number of strengthening webs 17 which may be provided behind the upper region of the lip 11 to assist in supporting the weight of the contents of the pouch 5.

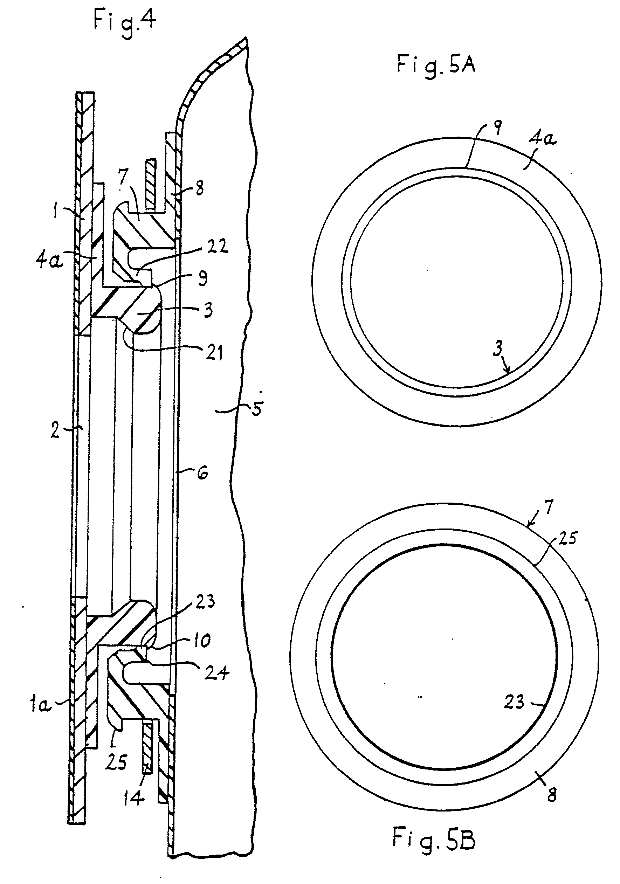

Figure 3 shows a further embodiment of the coupling ring 7 attached to a closed pouch 5, whereas in Figure 2 the pouch is shown as an open-ended drainable pouch. In the embodiment of Figure 3 the outer surface of the ring 7 includes an inclined region or ramp 18 such that when the two parts of the coupling have been engaged, the belt-attaching ring 14 can be moved axially up the ramp from a smaller diameter region 19 adjacent the pouch to a larger diameter region 20 adjacent the peripheral bead 15. In this latter position the ring 14 is a tight fit on the ring 7 and exerts a clamping force on the lip 11. The belt-attaching ring 14 is preferably made of more rigid material than the coupling rings 3 and 7.

Reference will now be made to the embodiment shown in Figures 4, 5A and 5B, wherein the same reference numerals are used for parts corresponding to those in the embodiment of Figure 1. Thus, a pad 1 has a stoma aperture 2 surrounded by a ring 3, which forms one part of the coupling and which in this embodiment is attached to the pad by an outwardly directed flange 4a. The inner surface of the ring 3 also includes an inclined seating 21 which can serve to locate a detachable ring (not shown), as is known in the art, where this is desirable in order more closely to match the aperture in the pad to the size of any particular stoma.

The other coupling part comprises a ring 7 attached to the pouch 5 about the aperture 6 by means of the outwardly directed flange 8. In this embodiment, the lip 22 provided to engage behind the bead 9 on the ring 3 extends in a direction generally parallel to the axis of the ring 7 and terminates in a generally square section end 23 of which one side 24 fits snugly against the outwardly extending wall portion 10 of the bead 9 on the ring 3.

As in the previous embodiment, the two parts of the coupling are engaged and disengaged by flexing of the lip 22 so that it can be moved over the bead 9 on the ring 3, and when the two parts are engaged the end 23 of the lip is squarely seated behind the bead 9 to form a secure joint.

The coupling may also be provided with other features of the previous embodiment, such as a pull tab on the ring 7 and also a rotatable and axially slidable clamping or belt-attaching ring 14 located behind the bead 25.

The coupling rings 3 and 7 are preferably made of thermoplastics material such as polyethylene and the pouch 5 and the surface of the pad 1 adjacent to the ring 3 are also made of suitable plastics materials. The coupling rings are respectively attached to the pouch and the pad by means of an adhesive or by means of a plastics welding technique.

The surface of the pad 1 which is to contact the skin of the abdomen is coated with an adhesive covered by a release paper 1a until it is required for use, the adhesive being of a type known in the art which causes minimum irritation to the skin of the user.

It will be understood that the precise nature of the plastics materials used for the various components of the ostomy appliance according to this invention will be determined by those skilled in the art from amongst those materials which are currently most suited for the purpose.

The coupling device of this invention is such that it is of little bulk and allows the pouch to be located closely adjacent the body of a user. It is also relatively free from crevices or like regions in which waste matter from the pouch may collect and this, in conjunction with the wiping action of the free end of the lip over the outer surface of the ring 3 attached to the pad as the pouch is removed, means that there is little likelihood of the outer surface of the ring 3 becoming soiled. The coupling is therefore lot only of reduced bulk but more acceptable to a user.

**Claims**

1. A coupling for an ostomy appliance comprising two generally cylindrical rings (3, 7) which can be interengaged concentrically, the inner ring (3) having means for attachment to an abdominal pad (1), and the outer ring (7) having means for attachment to an ostomy pouch (5), said inner ring including a radially outwardly directed peripheral bead (9) and said outer ring including a resilient lip (11, 12, 22) extending from the radially inner surface thereof whereby said lip becomes engaged behind said bead when the outer ring (7) is fitted over the inner ring (3) to make the coupling, characterised in that the outer ring (7) attached to the pouch (5) is fitted with a belt attaching ring (14) which is mounted on said outer ring (7) so as to be freely rotatable thereon and which is provided with attachment means (16) to which the ends of a belt fitted around the waist of a user may be secured.

2. An ostomy pouch (5) including an aperture (6) for the passage of the stoma and surrounded by a ring (7) forming the outer element of a concentric coupling (3, 7) for coupling the pouch to a pad (1) attachable to the abdomen of a user, about the stoma, wherein said ring (7) includes a resilient lip (11, 12, 22) extending from the radially inner surface of said ring and the free end of said lip faces towards the aperture in said pouch, characterised in that said ring (7) carries a freely rotatable belt attaching ring (14) mounted thereon and said belt attaching ring is provided with means (16) for the attachment of the ends of a body belt fitted around the waist of a user.

3. A device as claimed in claim 1 or 2, characterised in that the means for the attachment of the ends of a body belt comprises diametrically spaced apertured lugs (16).

4. A device as claimed in claim 1, 2 or 3, characterised in that said outer ring (7) is provided with an outwardly directed peripheral bead (15, 25) to assist in retaining the rotatable belt attaching ring (14).

5. A coupling as claimed in claim 1, characterised in that the rotatable belt attaching ring (14) is also axially slidable on the outer ring (7) to a position whereat it assists in clamping the coupling parts in the interengaged position.

6. An ostomy pouch as claimed in claims 2 and 4, characterised in that the rotatable belt attaching ring (14) is axially movable towards and away from the peripheral bead (15, 25).

**Patentansprüche**

1. Kupplung für eine Ostomieeinrichtung, die zwei allgemein zylindrische Ringe (3, 7) umfaßt, die konzentrisch miteinander in Eingriff gebracht werden können, wobei der innere Ring (3) Mittel zur Anbringung an einer abdominalen Konsole (1) und der äußere Ring (7) Mittel zum Anbringen an einem Ostomiebeutel (5) aufweist und wobei der innere Ring einen radial auswärts gerichteten Umfangswulst (9) und der äußere Ring eine federnde Lippe (11, 12, 22), die von der radialen Innenfläche des äußeren Ringes vorsteht, aufweist, wodurch die Lippe hinter dem Wulst eingreifen wird, wenn der äußere Ring (7) über den inneren Ring (3) gesetzt wird, um die Kupplung herzustellen, dadurch gekennzeichnet, daß der an dem Beutel (5) angebrachte äußere Ring (7) mit einem Riemenbefestigungsring (14) versehen ist, der an dem äußeren Ring (7) so montiert ist, daß er daran frei drehbar ist, und der mit Befestigungsmitteln (16) versehen ist, an denen die Enden eines um die Taille eines Benutzers gebundenen Riemens befestigt werden können.

2. Ostomiebeutel (5), der eine Öffnung (6) für den Durchgang des Stoma und einen diese umgebenden Ring (7) einschließt, wobei der Ring das äußere Element einer konzentrischen Kupplung (3, 7) zum Ankuppeln des Beutels an einer Konsole (1), die am Abdomen des Benutzers um das Stoma herum anbringbar ist, bildet, wobei der Ring (7) eine federnde Lippe (11, 12, 22) einschließt, die von der radialen Innenfläche des Ringes vorsteht und wobei das freie Ende der Lippe in Richtung des Loches in dem Beutel zeigt, dadurch gekennzeichnet, daß der Ring (7) einen frei drehbaren Riemenbefestigungsring (14) trägt, der darauf montiert ist, und daß der Riemenbefestigungsring mit Mitteln (16) zum Anbringen der Enden eines Körperriemens versehen ist, der um die Taille eines Benutzers gebunden ist.

3. Einrichtung nach Anspruch 1 oder 2, dadurch gekennzeichnet, daß die Mittel zum Anbringen der Enden eines Körperriemens diametral voneinander beabstandete, gelochte Ansätze (16) umfaßt.

4. Einrichtung nach Anspruch 1, 2 oder 3, dadurch gekennzeichnet, daß der äußere Ring (7) mit einem nach außen gerichteten Umfangswulst (15, 25) versehen ist, um beim Zurückhalten des drehbaren Riemenbefestigungsringes (14) zu unterstützen.

5. Kupplung nach Anspruch 1, dadurch gekennzeichnet, daß der drehbare Riemenbefestigungsring (14) auf dem äußeren Ring (7) ebenfalls axial verschiebbar ist in eine Stellung, in der er das Klemmen der Kupplungsteile in die Eingriffsstellung unterstützt.

6. Ostomiebeutel nach Anspruch 2 und 4, dadurch gekennzeichnet, daß der drehbare Riemenbefestigungsring (14) axial auf den Umfangswulst (15, 25) zu- und davon wegbewegbar ist.

**Revendications**

1. Accouplement pour un dispositif d'anus artificiel, comprenant deux bagues généralement cylindriques (3, 7), pouvant être disposées concentriquement l'une par rapport à l'autre, la bague intérieure (3) possédant un moyen de fixation à un coussin abdominal (1), et la bague extérieure (7) ayant un moyen de fixation à une poche pour anus artificiel (5), ladite bague intérieure comportant un bourrelet périphérique (9), dirigé radialement vers l'extérieur, et ladite bague extérieure comportant une lèvre élastique (11, 12, 22) s'étendant à partir de sa surface radialement intérieure, ce par quoi ladite lèvre s'engage derrière ledit bourrelet quand la bague extérieure (7) est ajustée sur la bague intérieure (3) pour réaliser l'accouplement, caractérisé en ce que la bague extérieure (7) fixée à la poche (5) est garnie d'une bague (14), pour fixation à une ceinture, et qui est montée sur ladite bague extérieure (7) de façon à pouvoir tourner librement sur elle, et qui est pourvue d'un moyen de fixation (16) par lequel on peut fixer en position les extrémités d'une ceinture installée autour de la taille d'un utilisateur.

2. Poche pour anus artificiel (5) comprenant une ouverture (6) pour le passage de l'abouchement, et entourée d'une bague (7) formant l'élément extérieur d'un accouplement concentrique (3, 7) destiné à accoupler la poche à un coussin (1) pouvant être fixé à l'abdomen d'un utilisateur, autour de l'abouchement, dans laquelle ladite bague (7) comprend une lèvre élastique (11, 12, 22) s'étendant à partir de la surface radialement intérieure de ladite bague, et l'extrémité libre de ladite lèvre regarde vers l'ouverture aménagée dans ladite poche, caractérisée en ce que ladite bague (7) supporte une bague (14), pouvant tourner librement et destinée à une fixation à une ceinture, montée sur la première bague, ladite bague de fixation à une ceinture étant pourvue d'un moyen (16) destiné à fixer les extrémités d'une ceinture corporelle placée autour de la taille d'un utilisateur.

3. Dispositif selon la revendication 1 ou 2, caractérisé en ce que le moyen pour fixation des extrémités d'une ceinture corporelle comprend des pattes ajourées (16), diamétralement opposées.

4. Dispositif selon la revendication 1, 2 ou 3, caractérisé en ce que ladite bague extérieure (7) est pourvue d'un bourrelet périphérique (15, 25) dirigé vers l'extérieur, pour aider à retenir la bague tournante (14) destinée à la fixation à une ceinture.

5. Accouplement selon la revendication 1, caractérisé en ce que la bague tournante de fixation à une ceinture (14) peut de même coulisser axialement sur la bague extérieure (7) jusqu'à une position dans laquelle elle facilite la fixation des éléments d'accouplement dans leur position en prise l'un par rapport à l'autre.

6. Poche pour anus artificiel selon les revendications 2 et 4, caractérisée en ce que la bague tournante de fixation à une ceinture (14) peut se déplacer axialement, vers le bourrelet périphérique (15, 25), et à partir de ce dernier.

Fig.1

Fig.3

Fig.2

Fig.4

Fig.5A

Fig.5B